# EUROPÄISCHE PATENTSCHRIFT

(11) **EP 4 178 649 B1**
(45) Veröffentlichungstag und Bekanntmachung des Hinweises auf die Patenterteilung: **22.10.2025**
(21) Anmeldenummer: 21745930.4
(22) Anmeldetag: 06.07.2021
(51) Int. Cl.: A61M 16/00

(54) **NOTFALL-BEATMUNGSGERÄT MIT ABNEHMBAREM GEMEINSAMEN DECKEL ZUR GLEICHZEITIGEN ABDECKUNG VON FILTER- UND BATTERIEFACH**
EMERGENCY VENTILATOR WITH REMOVABLE COMMON COVER FOR SIMULTANEOUSLY COVERING FILTER AND BATTERY COMPARTMENT
RESPIRATEUR AVEC COUVERCLE COMMUN AMOVIBLE POUR RECOUVRIR SIMULTANÉMENT UN FILTRE ET UN COMPARTIMENT DE BATTERIE

(30) Priorität: 13.07.2020 DE 102020118467
(43) Veröffentlichungstag der Anmeldung: 17.05.2023
(73) Patentinhaber: Hamilton Medical AG, 7402 Bonaduz (CH)
(72) Erfinder: HEPTING, Daniel, 7026 Maladers (CH)
(74) Vertreter: Ruttensperger Lachnit Trossin Gomoll
(86) Internationale Anmeldenummer: PCT/EP2021/068623
(87) Internationale Veröffentlichungsnummer: WO 2022/013004

(56) Entgegenhaltungen:
- US-A1- 2011 197 882
- US-A1- 2019 366 025
- US-B2- 8 297 279

## Beschreibung

Die vorliegende Erfindung betrifft ein Notfall-Beatmungsgerät zur notfallmedizinischen künstlichen Beatmung von Patienten, umfassend
- ein Gehäuse mit einer Umgebungsluft-Ansaugöffnung und einer Beatmungsgas-Ausgabeöffnung,
- ein Gebläse, welches dazu ausgebildet und im Gehäuse angeordnet ist, um Umgebungsluft von der Umgebungsluft-Ansaugöffnung zur Beatmungsgas-Ausgabeöffnung zu fördern,
- einen Luftfilter, welcher zur Reinigung von angesaugter Umgebungsluft ausgebildet ist und im Gehäuse im Strömungsweg der Umgebungsluft stromabwärts der Umgebungsluft-Ansaugöffnung angeordnet ist, und
- einem Energiespeicher zur Versorgung des Gebläses mit Energie für dessen Betrieb,

wobei der Luftfilter durch eine mittels eines Gehäusedeckels verschlossene, aber öffenbare Gehäuseöffnung zugänglich und bestimmungsgemäß austauschbar im Gehäuse aufgenommen ist, und
wobei der Energiespeicher durch eine mittels eines Gehäusedeckels verschlossene, aber öffenbare Gehäuseöffnung zugänglich und bestimmungsgemäß austauschbar im Gehäuse aufgenommen ist.

Als ein solches Notfall-Beatmungsgerät sind bekannt das Beatmungsgerät mit der Bezeichnung "EVE_{IN}" der Fritz Stephan GmbH in Gackenbach (DE) sowie das Beatmungsgerät mit der Bezeichnung "Falco 202 Evo" der italienischen Firma Siare Engineering International Group s. r. l. in Valsamoggia (IT).

Aus der US 2011/0197882 A1 ist ein tragbares Beatmungsgerät bekannt, wobei Luftfilter hinter einem abnehmbaren Gehäusedeckel verborgen sind und wobei ein abnehmbarer Batteriepack in einer Gehäusemulde des Gerätegehäuses angeordnet ist.

Eine Außenfläche des Batteriepacks bildet bei in der Gehäusemulde angeordnetem Batteriepack eine Außenfläche des Beatmungsgeräts.

Notfall-Beatmungsgeräte, unter anderem auch als "intensivmedizinische Beatmungsgeräte" bezeichnet, dienen der schnellen Versorgung eines Patienten mit Beatmungsgas außerhalb einer klinischen Umgebung, also etwa an einem Unfallort oder/und während eines Transports eines Patienten. Selbstverständlich können Notfall-Beatmungsgeräte auch in einer klinischen Umgebung eingesetzt werden, jedoch stehen in Kliniken häufig leistungsfähigere Beatmungsgeräte als Notfall-Beatmungsgeräte zur Verfügung.

Als außerhalb einer klinischen Umgebung einsetzbare Beatmungsgeräte weisen Notfall-Beatmungsgeräte einen eigenen Energiespeicher auf, welcher zumindest für eine gewisse Dauer einen Betrieb des Notfall-Beatmungsgeräts unabhängig von einer Netzversorgung ermöglicht. Außerdem sind Notfall-Beatmungsgeräte hinsichtlich ihrer Größe und ihres Gewichts als tragbare Beatmungsgeräte ausgelegt, so dass sie von einem Notfall-Mediziner, etwa einen an einen Unfallort gerufenen Notarzt, nur mit eigener Muskelkraft auch über Strecken von mehreren Dutzend Metern ohne übermäßige körperliche Belastung bewegt werden können.

Ohne ergänzende Sondergasvorräte, wie etwa einen Sauerstoffvorrat, sind Notfall-Beatmungsgeräte aufgrund ihres Gebläses darauf ausgelegt, wenigstens Umgebungsluft als Beatmungsgas verabreichen zu können. Der Umgebungsluft kann bei Bedarf ein von der Umgebungsluft verschiedenes Sondergas, im häufigsten Fall reiner Sauerstoff, aber auch anästhetische oder/und therapeutische Gase und Gasgemische beigemischt werden. Hierzu weisen Notfall-Beatmungsgeräte üblicherweise eine Anschlussformation zum Anschluss eines Sondergasvorrats auf.

Umgebungsluft, die vom Notfall-Beatmungsgerät als Beatmungsgas angesaugt wird, kann verschmutzt sein. Man denke hier beispielsweise an notfallmedizinisch zu versorgende Unfallopfer auf Baustellen oder in sonstiger staubiger oder sandiger Umgebung.

Um einem an Ort und Stelle künstlich zu beatmenden Patienten ausreichend sauberes Beatmungsgas verabreichen zu können, ist im Strömungsweg der Umgebungsluft von der Ansaugöffnung bis zum Patienten ein Luftfilter angeordnet, welcher abhängig von seinen Filtereigenschaften Schmutz aus der angesaugten Umgebungsluft entfernt, sodass der Schmutz den Patienten nicht erreicht. Im Gehäuse des Notfall-Beatmungsgeräts ist der Luftfilter zwischen der Umgebungsluft-Ansaugöffnung und der Beatmungsgas-Ausgabeöffnung angeordnet.

Sowohl der Luftfilter als auch der Energiespeicher haben eine endliche Gebrauchsdauer, welche erheblich kürzer ist als die Betriebslebensdauer des Notfall-Beatmungsgeräts. Sie müssen dementsprechend häufig getauscht werden. Für Notfall-Beatmungsgeräte ist es wesentlich, dass sie in der kürzest möglichen Zeit einsatzbereit gemacht werden können. Während eines Tausches des Luftfilters oder des Energiespeichers ist das Notfall-Beatmungsgerät nicht einsatzbereit.

Es ist daher Aufgabe der vorliegenden Erfindung, das oben angegebene Notfall-Beatmungsgerät derart weiterzubilden, dass es im Falle eines notwendigen Tausches des Luftfilters oder/und des Energiespeichers für eine möglichst kurze Zeit ausfällt.

Die vorliegende Erfindung löst diese Aufgabe an einem Notfall-Beatmungsgerät mit den eingangs genannten Merkmalen dadurch, dass der Luftfilter und der Energiespeicher durch eine gemeinsame Gehäuseöffnung zugänglich sind, wobei die gemeinsame Gehäuseöffnung durch einen gemeinsamen Gehäusedeckel wahlweise verschließbar und öffenbar ist.

Anders als bei den oben genannten Notfall-Beatmungsgeräten, die den Luftfilter und den Energiespeicher an unterschiedlichen Orten im jeweiligen Gehäuse aufweisen, sodass der Luftfilter und der Energiespeicher durch unterschiedliche Gehäuseöffnungen zugänglich sind, wobei die unterschiedlichen Gehäuseöffnungen durch gesonderte Gehäusedeckel verschließbar sind, sind bei dem vorliegenden Notfall-Beatmungsgerät der Luftfilter und der Energiespeicher durch ein und dieselbe gemeinsame Gehäuseöffnung zugänglich, wobei die gemeinsame Gehäuseöffnung durch einen einzelnen gemeinsamen Gehäusedeckel verschließbar ist.

Dies lässt es dann, wenn sowohl der Luftfilter als auch der Energiespeicher getauscht werden müssen, ausreichen, nur einen einzigen gemeinsamen Gehäusedeckel abzunehmen, um sowohl Luftfilter als auch Energiespeicher zugänglich zu machen und tauschen zu können. Auch muss nur ein einziger gemeinsamer Gehäusedeckel nach dem Tausch wieder an der gemeinsamen Gehäuseöffnung angeordnet werden. Dass dann, wenn nur ein Bauteil aus Luftfilter und Energiespeicher getauscht werden muss, stets beide Bauteile zugänglich sind, ist nicht nur unschädlich, denn bereits das Tauschen eines dieser Bauteile setzt das Notfall-Beatmungsgerät außer Betrieb, sondern sogar vorteilhaft. Denn dadurch, dass nur ein gemeinsamer Gehäusedeckel abzunehmen ist, ist eine zeitraubende, unerwünschte Verwechslung von unterschiedlichen Bauteilen zugeordneten unterschiedlichen Gehäusedeckeln unmöglich, sodass durch die Verwendung des gemeinsamen Gehäusedeckels über viele Tauschvorgänge hinweg auch die mittlere Ausfallzeit des Notfall-Beatmungsgeräts verkürzt wird.

Der Energiespeicher ist in der Regel ein elektrischer Energiespeicher, wie eine Batterie oder ein wiederaufladbarer Akkumulator oder kurz "Akku".

Ein nachfolgend als "Verschlusszustand" bezeichneter Zustand des Notfall-Beatmungsgeräts bezeichnet einen Zustand, in welchem der gemeinsame Gehäusedeckel die gemeinsame Gehäuseöffnung verschließt, also abdeckt, sodass von außen hinter der Gehäuseöffnung angeordnete Bauteile und Bauteilabschnitte durch den gemeinsamen Gehäusedeckel abgeschirmt sind und somit nicht zugänglich sind.

Um ein unbeabsichtigtes Entfernen des gemeinsamen Gehäusedeckels von der gemeinsamen Gehäuseöffnung zu vermeiden, ist der gemeinsame Gehäusedeckel bevorzugt an dem übrigen, die Gehäuseöffnung aufweisenden Restgehäuse verriegelbar. Zur sicheren Verriegelung weist der gemeinsamen Gehäusedeckel bevorzugt eine Verriegelungsformation auf, welche in Formschlusseingriff mit einer gehäusefesten Verriegelungsgegenformation bringbar ist. Ein Formschlusseingriff sorgt mittels einer durch ihn bewirkten körperlichen Bewegungsblockade des gemeinsamen Gehäusedeckels relativ zur gemeinsamen Gehäuseöffnung für eine besonders sichere Verriegelung des gemeinsamen Gehäusedeckels an der gemeinsamen Gehäuseöffnung.

Nachfolgend wird bei den Formationen "Gehäusedeckel" und "Gehäuseöffnung" das Attribut "gemeinsam" weggelassen, da jede weitere Nennung des Gehäusedeckels und der Gehäuseöffnung den gemeinsamen Gehäusedeckel bzw. die gemeinsame Gehäuseöffnung bezeichnet.

Das übrige Gehäuse ohne den davon abgenommenem Gehäusedeckel wird nachfolgend auch als "Restgehäuse" bezeichnet.

"Gehäusefest" bedeutet dabei nicht notwendigerweise unmittelbar am Gehäuse ausgebildet, wenngleich dies auch von dem Begriff "gehäusefest" umfasst ist. "Gehäusefest" bedeutet "vom Gehäuse bzw. Restgehäuse, außer zu etwaigen Reparaturzwecken, bestimmungsgemäß nicht trennbar bzw. nicht entfernbar".

Eine Formation aus Verriegelungsformation und Verriegelungsgegenformation kann ein Vorsprung sein, welche in die dann als Ausnehmung ausgebildete jeweilige andere Formation aus Verriegelungsformation und Verriegelungsgegenformation unter Bildung eines Formschlusseingriffs einragen kann.

Grundsätzlich kann die Verriegelungsformation starr am Gehäusedeckel angeordnet sein und kann die Verriegelungsgegenformation zwischen einer Verriegelungsstellung und einer Freigabestellung beweglich am Restgehäuse angeordnet sein. In der Verriegelungsstellung befindet sich die Verriegelungsformation in Formschlusseingriff mit der Verriegelungsgegenformation, in der Freigabestellung dagegen nicht. Um in einer vorteilhaften Weiterbildung der vorliegenden Erfindung zu ermöglichen, den Gehäusedeckel mit einer Hand und noch stärker bevorzugt mit einer einzigen flüssigen Bewegung vom Restgehäuse abzunehmen, ist jedoch bevorzugt die Verriegelungsformation zwischen einer Verriegelungsstellung und einer Freigabestellung beweglich am Gehäusedeckel vorgesehen. Auch eine Betätigungsformation für einen Handangriff zur Betätigung der Verriegelungsformation zwischen der Verriegelungsstellung und der Freigabestellung ist bevorzugt am Gehäusedeckel vorgesehen.

Im Hinblick auf einen möglichst sicheren und festen Verschluss der Gehäuseöffnung durch den Gehäusedeckel bei gleichzeitig möglicher einhändiger Bedienung des Gehäusedeckels zu seiner Entfernung von der Gehäuseöffnung und zu seiner Anordnung an der Gehäuseöffnung sowie seiner Verriegelung dort ist bevorzugt vorgesehen, dass der Gehäusedeckel ein im Verschlusszustand des Notfall-Beatmungsgeräts relativ zum übrigen Gehäuse unbewegliches Deckelbauteil und ein relativ zum übrigen Gehäuse bewegliches Verriegelungsbauteil aufweist. Das Verriegelungsbauteil trägt die oben genannte Verriegelungsformation, und bevorzugt ebenfalls die oben genannte Betätigungsformation, und ist bevorzugt beweglich zwischen der Verriegelungsstellung, in welcher die Verriegelungsformation des Verriegelungsbauteils durch Formschlusseingriff mit der gehäusefesten Verriegelungsgegenformation am übrigen Beatmungsgerät den Gehäusedeckel gegen ein Entfernen von der Gehäuseöffnung verriegelt, und der Freigabestellung, in welcher das Verriegelungsbauteil ein Entfernen des Gehäusedeckels von der Gehäuseöffnung gestattet.

Um die zur Bereitstellung des Gehäusedeckels benötigte Anzahl an Bauteilen möglichst gering zu halten, ist das Verriegelungsbauteil bevorzugt relativ zum Deckelbauteil beweglich am Deckelbauteil angeordnet und gehaltert.

Grundsätzlich kann das Verriegelungsbauteil relativ zum Deckelbauteil translatorisch zwischen der Verriegelungsstellung der Freigabestellung beweglich sein. Da jedoch in der Regel der Gehäusedeckel translatorisch von der Gehäuseöffnung entfernt wird ist es für eine einhändige Bedienung des Gehäusedeckels bei gleichzeitig größtmöglicher Bediensicherheit gegen eine unerwünschte Fehlbedienung vorteilhaft, wenn das Verriegelungsbauteil relativ zum Deckelbauteil um eine Verriegelungsachse drehbar am Deckelbauteil gelagert ist. Bevorzugt ist der Gehäusedeckel längs der Verriegelungsachse von der Gehäuseöffnung abhebbar. Dann kann der die Gehäuseöffnung verschließende und verriegelte Gehäusedeckel durch Drehen des Verriegelungsbauteils um die Verriegelungsachse entriegelt und ohne Veränderung des Handangriffs an das Verriegelungsbauteil längs der Verriegelungsachse von der Gehäuseöffnung weg entfernt werden.

Grundsätzlich können die Verriegelungsformation und die Verriegelungsgegenformation jeweils ein Gewinde sein, welche in der Verriegelungsstellung miteinander in Schraubeingriff stehen und in der Freigabestellung nicht. Zwar bietet ein Schraubeingriff eine besonders sichere Verriegelung. Jedoch benötigen sein Lösen und seine Wiederherstellung nicht unerhebliche Zeit. Bevorzugt bilden daher die Verriegelungsformation und die Verriegelungsgegenformation einen Bajonettverschluss. Eine Formation aus Verriegelungsformation und Verriegelungsgegenformation weist daher wenigstens einen bezüglich der Verriegelungsachse radialen Vorsprung auf und die jeweils andere Formation weist eine den Vorsprung aufnehmende Ausnehmung mit einem - bezogen auf die Verriegelungsachse - axialen Abschnitt und wenigstens einer in Umfangsrichtung um die Verriegelungsachse verlaufenden, vom Vorsprung in der Verriegelungsstellung hintergriffenen Umfangsrand auf. Der Rand kann eine Flanke eines Umfangsabschnitts der Ausnehmung sein. Bei Annäherung des Gehäusedeckels an die Gehäuseöffnung längs der Verriegelungsachse kann der Vorsprung im axialen Abschnitt relativ zur Ausnehmung axial gleiten bis der Umfangsrand axial den Vorsprung passiert hat. Dann kann der Vorsprung zur Verstellung in die Verriegelungsstellung durch Drehen des Verriegelungsbauteils um die Verriegelungsachse längs des Umfangsrandes gleiten, wobei er diesen körperlich hintergreift. Dadurch ist eine Bewegung des Gehäusedeckels in axialer Richtung von der Gehäuseöffnung weg formschlüssig verhindert.

Da der Gehäusedeckel einen Zugang zum Luftfilter schafft, welcher im Strömungsweg der Umgebungsluft von der Umgebungsluft-Ansaugöffnung bis zur Beatmungsgas-Auslassöffnung angeordnet ist, umfasst der Gehäusedeckel zur Erzielung einer vorteilhaft kompakten Bauweise des Notfall-Beatmungsgeräts bevorzugt die Umgebungsluft-Ansaugöffnung. Dadurch ist es außerdem möglich, den Luftfilter nahe des Gehäusedeckels, bevorzugt unmittelbar dahinter, stromaufwärts des Gebläses anzuordnen, so dass das Gebläse nur von gereinigter Luft durchströmt wird. Dies erhöht die Standzeit des Gebläses.

Grundsätzlich kann die Umgebungsluft-Ansaugöffnung im Deckelbauteil angeordnet sein. Für eine möglichst sichere Betätigung des Verriegelungsbauteils auch bei sehr schlechten Lichtverhältnissen ist dieses bevorzugt verhältnismäßig groß ausgebildet, sodass es auch durch Tasten leicht und schnell auffindbar ist. Daher kann die Umgebungsluft-Ansaugöffnung ohne weiteres das Verriegelungsbauteil durchsetzen.

Dann, wenn die Umgebungsluft-Ansaugöffnung das Verriegelungsbauteil durchsetzt, durchsetzt bevorzugt die Verriegelungsachse die Umgebungsluft-Ansaugöffnung, sodass sich bei einer Drehung des Verriegelungsbauteils um die Verriegelungsachse die Lage der Umgebungsluft-Ansaugöffnung relativ zum Restgehäuse möglichst wenig geändert. Aus diesem Grunde ist die Umgebungsluft-Ansaugöffnung besonders bevorzugt zentrisch am Verriegelungsbauteil angeordnet und wird zentrisch von der Verriegelungsachse durchsetzt. Im Falle einer bevorzugten kreisrunden Umgebungsluft-Ansaugöffnung ändert sich ihre Lage bei Drehung des Verriegelungsbauteils um die Verriegelungsachse nicht, sodass das Notfall-Beatmungsgerät noch während des Entriegelns des Gehäusedeckels weiter einsatzbereit ist.

Gemäß einer konstruktiv bevorzugten Ausführungsform kann das Deckelbauteil zur drehbaren Lagerung des Verriegelungsbauteils einen zur Verriegelungsachse koaxialen Lagerabschnitt aufweisen, welcher die Umgebungsluft-Ansaugöffnung umgibt, und welcher von einem sich längs der Verriegelungsachse erstreckenden und zur Verriegelungsachse koaxialen Lagergegenabschnitt des Verriegelungsbauteils umgeben ist. Dann kann der Lagerabschnitt als eine Art Achsbauteil das Verriegelungsbauteil um die Verriegelungsachse drehbar tragen. Zur Erzielung einer vorteilhaft großen Lagerlänge kann der Lagerabschnitt längs der Verriegelungsachse vom übrigen Deckelbauteil abstehen.

Um zusätzliche Filter zur Reinigung der angesaugten Umgebungsluft oder/und ein Messinstrument zur Erfassung physikalischer oder/und chemischer Eigenschaften der angesaugten Umgebungsluft, wie etwa Temperatur, Belastung mit vorbestimmten Schweb- oder Inhaltsstoffen oder Zusammensetzung, am Notfall-Beatmungsgerät anordnen zu können, weist der Lagerabschnitt auf seiner von dem Lagergegenabschnitt wegweisenden, bezüglich der Verriegelungsachse radial inneren Seite eine Befestigungsformation, vorzugsweise ein Gewinde, insbesondere Innengewinde, oder ein Teil einer weiteren Bajonettverriegelung, auf, an welcher Befestigungsformation der zusätzliche Filter oder/und das Messinstrument befestigt werden können.

Bevorzugt dient der Gehäusedeckel nicht nur zum Verschließen der Gehäuseöffnung, sondern er trägt auch zur Lagefixierung von hinter dem Gehäusedeckel im Verschlusszustand im Gehäuse aufgenommenen Funktionsbauteilen bei. Daher weist der Gehäusedeckel gemäß einer bevorzugten Weiterbildung einen im Verschlusszustand in das Innere des Gehäuses weisenden Deckel-Filter-Positionierabschnitt auf, welcher im Verschlusszustand bei betriebsbereitem Notfall-Beatmungsgerät im Zusammenwirken mit wenigstens einem gehäusefesten Gehäuse-Filter-Positionierabschnitt eine Filterkartusche zur Filterung von Umgebungsluft in ihrer betriebsbereiten Position sichert.

Zusätzlich oder alternativ kann der gemeinsame Gehäusedeckel einen im Verschlusszustand in das Innere des Gehäuses weisenden Deckel-Speicher-Positionierabschnitt aufweisen, welcher im Verschlusszustand bei betriebsbereitem Notfall-Beatmungsgerät im Zusammenwirken mit wenigstens einem gehäusefesten Gehäuse-Speicher-Positionierabschnitt einen Energiespeicherkörper in seiner betriebsbereiten Position sichert. Der Energiespeicherkörper kann die oben genannte Batterie oder der wiederaufladbare Akku sein.

Bevorzugt befinden sich die genannten gehäuseseitigen Positionierabschnitte und die zugeordneten deckelseitigen Positionierabschnitte im Verschlusszustand in Anlageeingriff mit dem jeweils positionierten Bauteil: Filterkartusche und Energiespeicherkörper, wobei auch ein Formschlusseingriff zwischen einem Positionierabschnitt und dem positionierten Bauteil nicht ausgeschlossen sein soll.

Die Filterkartusche weist bevorzugt ein Filtergehäuse und einen im Filtergehäuse aufgenommenen Filter, insbesondere HEPA-Filter, auf, wobei die Filterkartusche als ein Bauteil bei geöffneter Gehäuseöffnung aus dem Restgehäuse entnehmbar ist bzw. in das Restgehäuse einsetzbar ist. Ebenso ist bevorzugt der Energiespeicherkörper ein einziger Energiespeicherkörper, wenngleich nicht ausgeschlossen sein soll, dass ein hoher Energiebedarf eines Notfall-Beatmungsgeräts mehr als einen Energiespeicherkörper erfordert.

Die Filterkartusche weist eine im betriebsbereiten Zustand des Notfall-Beatmungsgeräts durch die Umgebungsluft-Ansaugöffnung erreichbare Umgebungsluft-Einlassöffnung auf. Diese kann zur Verhinderung eines Eintritts großer Schmutzpartikel mit einem Schutzgitter versehen sein. Das Schutzgitter kann, etwa bei spritzgusstechnischer Ausbildung der Filterkartusche, einstückig mit einem Bauteil des Kartuschengehäuses ausgebildet sein. Da es, wie oben bereits angedeutet, in manchen Beatmungssituationen erforderlich sein kann, zusätzlich zur Umgebungsluft ein von dieser abweichendes Sondergas zu verabreichen, kann die Filterkartusche zusätzlich zur Umgebungsluft-Einlassöffnung eine Sondergas-Anschlussformation zum Anschluss einer Sondergasversorgung aufweisen.

Damit die Sondergas-Anschlussformation der Filterkartusche durch die Umgebungsluft-Ansaugöffnung des Gehäusedeckels leicht erreichbar ist, ist es vorteilhaft, wenn die Sondergas-Anschlussformation mit - bezogen auf eine die Umgebungsluft-Ansaugöffnung zentral durchsetzend gedachte virtuelle Achse - radialem Abstand von einem Rand der Umgebungsluft-Ansaugöffnung angeordnet ist. Bevorzugt sind daher die Umgebungsluft-Einlassöffnung und die Sondergas-Anschlussformation zueinander koaxial angeordnet. Besonders bevorzugt ist die Sondergas-Anschlussformation im betriebsbereiten Verschlusszustand auch koaxial zur Verriegelungsachse angeordnet.

Zahlreiche Notfall-Beatmungsgeräte des Standes der Technik zeigen eine komplexe Außengestalt mit zahlreichen relativ zueinander angewinkelten Flächen. Eine solche Gestalt kann in der Aufregung und Hektik eines Notfalleinsatzes hinderlich sein, da sich Schläuche und Drähte an Ecken und in Spalten an derart komplexen Gestalten verfangen können. Bevorzugt hat daher das Gehäuse des vorliegenden Notfall-Beatmungsgeräts eine einfache Gehäusegestalt, vorzugsweise mit einer prismatischen oder/und zylindrischen Grundform. Somit hat das Gehäuse bevorzugt zwei zueinander im Wesentlichen parallele Stirnseiten und eine die beiden Stirnseiten miteinander verbindende Mantelfläche. Die Mantelfläche läuft um eine virtuelle, die Stirnseiten miteinander verbindende Prismenachse um. Die Mantelfläche kann dabei polyedrisch mit in Umlaufrichtung um die Prismenachse aufeinander folgenden ebenen Flächen ausgestaltet sein. Zur Vermeidung von Verletzungen sind die Verbindungsbereiche zwischen zwei in Umlaufrichtung unmittelbar benachbarten ebenen Mantelflächenabschnitten abgerundet. Bevorzugt weist der Krümmungsradius eines solchen Verbindungsabschnitts wenigstens 0,5 cm auf. Die Krümmungsachse ist vorzugsweise parallel zur Prismenachse. Die Mantelfläche kann ebenso zylindrisch ausgebildet sein, wobei die Querschnittsfläche der zylindrischen Grundform kreisförmig oder elliptisch sein kann.

Die Mantelfläche kann auch sowohl prismatisch als auch zylindrisch sein, wenn sie etwa längs eines ersten Umfangsabschnitts polyedrisch und längs eines daran anschließenden zweiten Umfangsabschnitts zylindrisch bzw. teilzylindrisch ausgebildet ist.

Das die Mantelfläche aufweisende Gehäusebauteil ist bevorzugt ein rohrförmiges Gehäusebauteil, wobei dessen Rohrachse die Prismenachse ist. Das rohrförmige Gehäusebauteil ist aus Gründen geringen Gewichts und guter Wärmeleitung bevorzugt aus einem Leichtmetall, wie einer Aluminium- oder Magnesiumlegierung gebildet, kann zusätzlich oder alternativ auch aus einer Kupferlegierung gebildet sein, wie beispielsweise Messing oder Bronze, oder auch aus einer kupferhaltigen Legierung. Abweichend davon kann das rohrförmige Gehäusebauteil aus Kunststoff, insbesondere aus thermoplastischem Kunststoff hergestellt sein. Zur Erhöhung der Wärmeleitfähigkeit kann der Kunststoff mit Partikeln gefüllt sein, welche die Wärmeleitfähigkeit der Mischung gegenüber der ungefüllten Kunststoffmatrix erhöhen. Ein solches Füllmaterial ist beispielsweise Bornitrid.

Aus Gründen erhöhter Stabilität ist das rohrförmige Gehäusebauteil bevorzugt fügestellenfrei gefertigt, etwa als Strangpressbauteil oder als Strangextrusionsbauteil.

Bevorzugt bildet der Gehäusedeckel eine Stirnseite des prismatischen oder/und zylindrischen Gehäuses. So kann die Mantelfläche des Gehäuses für die Anordnung des Gehäusedeckels am Restgehäuse genutzt werden. Selbstverständlich kann alternativ die Gehäuseöffnung auch in einer bestehenden Gehäusewand durch Ausschneiden von Wandmaterial erzeugt werden. Dieser Aufwand ist jedoch nicht nötig, wenn man eine Gehäuseöffnung nutzt, die bei der Herstellung eines Gehäuses mit einer prismatischen oder/und zylindrischen Grundform ohnehin entsteht, wie etwa eine stirnseitige Öffnung.

Zur besseren Orientierung einer das Notfall-Beatmungsgerät bedienenden Person auch bei schlechten Lichtverhältnissen ist es vorteilhaft, wenn alle ein Gas in das Gehäuse einleitenden und Gas aus dem Gehäuse ausleitenden Anschlussformationen oder/und Öffnungen kumuliert an nur einer Stirnseite oder verteilt an beiden Stirnseiten des prismatischen oder/und zylindrischen Gehäuses angeordnet sind. Außerdem können so vorstehende und deshalb beschädigungsgefährdete Anschlussformationen, wie etwa Anschlussstutzen, an der Mantelfläche vermieden werden. Mögliche Anschlussformationen können Stutzen, insbesondere Gewindestutzen, Schnellkupplungen, Gewindeausnehmungen und dergleichen sein.

Zur Bedienung und Steuerung des Notfall-Beatmungsgeräts weist es bevorzugt eine Eingabe-/Ausgabe-Vorrichtung auf, mit welcher Daten oder/und Steuerbefehle in das Notfall-Beatmungsgerät eingegeben werden können und mit welcher der bedienenden Person Informationen über den Betrieb des Notfall-Beatmungsgeräts angezeigt werden können. Die Eingabe-/Ausgabe-Vorrichtung weist daher bevorzugt eine Anzeigevorrichtung auf, wie etwa einen Bildschirm, und weist wenigstens eine Schaltvorrichtung, wie beispielsweise einen Tastschalter oder/und einen Kippschalter oder/und einen Drehschalter, auf. Der Bildschirm kann bevorzugt ein Touchscreen sein, sodass die Menge an fest am Notfall-Beatmungsgerät installierten Tasten, also Tastschaltern, gering gehalten werden kann. Die Eingabe-/Ausgabe-Vorrichtung sowie die weitere Steuerungs- und Auswerte-Elektronik des Notfall-Beatmungsgeräts sind ebenfalls durch den Energiespeicher mit Energie versorgt.

Stärker bevorzugt weist das prismatische oder/und zylindrische Gehäuse die Eingabe-/Ausgabe-Vorrichtung mit der Anzeigevorrichtung und der wenigstens einen Schaltvorrichtung im Bereich seiner Mantelfläche, vorzugsweise nur im Bereich seiner Mantelfläche, auf.

Bevorzugt ist ein Großteil, also mehr als die Hälfte, bevorzugt mehr als 70 %, der von außen wahrnehmbaren Gehäusewand aus stoßfestem Material, wie etwa aus Metall oder aus Kunststoff, insbesondere gefülltem Kunststoff, hergestellt, um das Notfall-Beatmungsgerät ausreichend robust für die oft raue Umgebung und Handhabung bei Notfall-Einsätzen auszurüsten. Aus Gewichtsgründen ist eine metallische Gehäuse wand bevorzugt aus einer Aluminium- oder Magnesiumlegierung gebildet. Um dennoch Stöße, die etwa bei heftigem Abstellen des Notfall-Beatmungsgeräts entstehen können, nicht ungedämpft zur Elektronik im Inneren des Notfall-Beatmungsgeräts zu übertragen, weist das Notfall-Beatmungsgerät vorzugsweise an seiner Außenfläche wenigstens ein stoßdämpfendes Element auf. Bevorzugt ist das stoßdämpfendes Element aus einem elastomeren Kunststoff, wie etwa Kautschuk oder Gummi, insbesondere Silikongummi, gebildet, welcher einen erheblich geringeren Elastizitätsmodul aufweist als das robuste Material, das einen Großteil der Gehäusewand bildet. Wegen der vorteilhaften Möglichkeiten zur urformenden Formgebung ist ein thermoplastisches Elastomer ein vorteilhafter elastomerer Kunststoff. Bevorzugt ist das wenigstens eine stoßdämpfende Element an der Außenfläche des Gehäusedeckels angeordnet. Im Falle der bevorzugten Ausgestaltung des Gehäusedeckels als vollständige Stirnfläche eines prismatischen oder/und zylindrischen Gehäuses ist das wenigstens eine stoßdämpfende Element vorzugsweise in Umlaufrichtung um die Prismenachse am Gehäusedeckel vorgesehen, besonders bevorzugt vollständig geschlossen um die Prismenachse umlaufend.

Die vorliegende Erfindung wird nachfolgend anhand der beiliegenden Figuren näher erläutert. Es stellt dar:
- Figur 1: eine perspektivische Explosionsansicht eines erfindungsgemäßen Notfall-Beatmungsgeräts,
- Figur 2: eine Längsschnittansicht durch das erfindungsgemäße Notfall-Beatmungsgerät von Figur 1, mit einer Schnittebene parallel zu den Flächen 14b und 14d in Fig. 1,
- Figur 3: eine Draufsicht auf die von einem abnehmbaren Gehäusedeckel gebildete eine Stirnseite des Notfall-Beatmungsgeräts der Figuren 1 und 2,
- Figur 4: eine Draufsicht auf die andere, entgegengesetzte Stirnseite des Notfall-Beatmungsgeräts der Figuren 1 und 2,

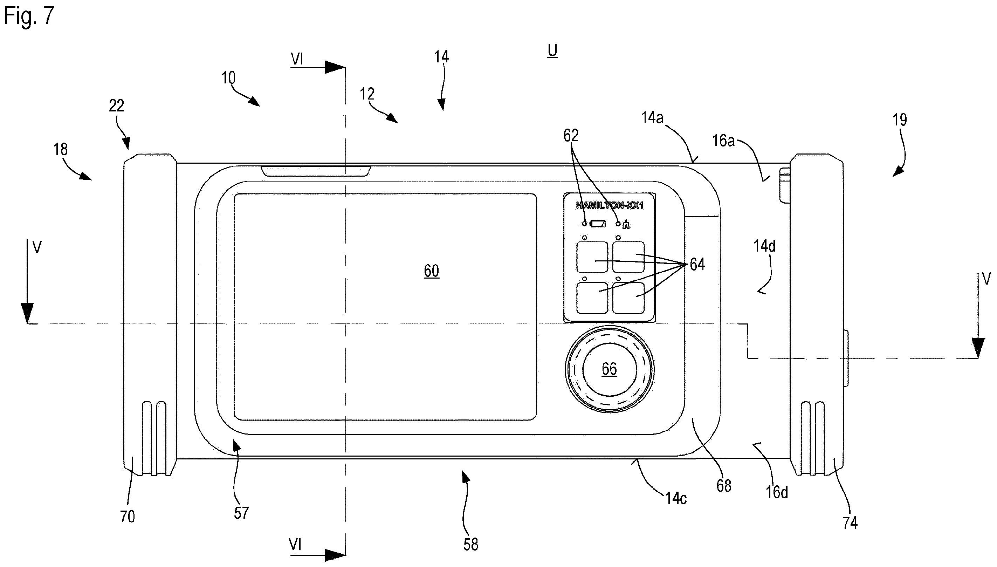

- Figur 5: eine Längsschnittansicht entlang der Schnittfläche V-V von Figur 7,
- Figur 6: eine Querschnittansicht entlang der zur Prismenachse P orthogonalen Schnittebene VI-VI von Figur 7, und
- Figur 7: eine Draufsicht auf die ebene Vorderfläche 14d mit der Eingabe-/AusgabeVorrichtung 58 des Notfall-Beatmungsgeräts von Figur 1.

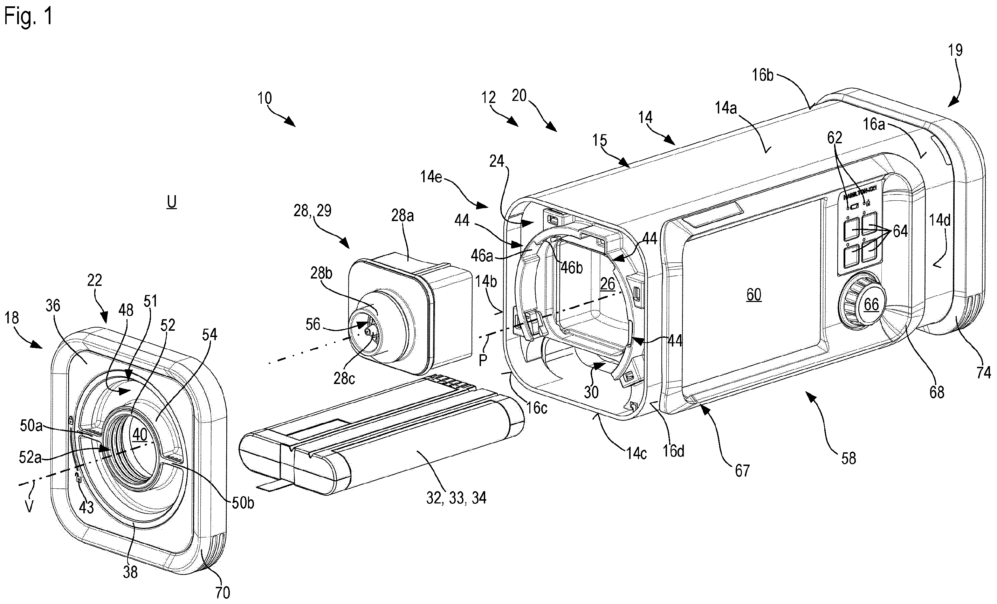

In Figur 1 ist eine erfindungsgemäße Ausführungsform eines Notfall-Beatmungsgeräts allgemein mit 10 bezeichnet. Das Notfall-Beatmungsgerät 10 umfasst ein Gehäuse 12 mit prismatischer Grundform, vorliegend mit quaderartiger Grundform.

Die Mantelfläche 14 des Gehäuses 12 umfasst vier ebene Flächenanteile 14a, 14b, 14c und 14d, von welchen jeweils in Umlaufrichtung um die Prismenachse P aufeinanderfolgende ebene Flächenanteile 14a, 14b, 14c und 14d zueinander orthogonal orientiert sind. Alle ebenen Flächenanteile 14a, 14b, 14c und 14d sind zur Prismenachse P parallel. Die ebenen Flächenanteile 14a, 14b, 14c und 14d sind durch viertelzylindrische Flächenanteile 16a, 16b, 16c und 16d miteinander bevorzugt fügestellenfrei verbunden. Die einzelnen Zylinderachsen der viertelzylindrischen und damit gekrümmten Flächenanteile 16a, 16b, 16c und 16d sind parallel zur Prismenachse P. Bevorzugt ist das die Mantelfläche 14 aufweisende Gehäusebauteil 15 ein extrudiertes Aluminiumrohr.

An der dem Betrachter von Figur 1 zugewandten Stirnseite 18 des Gehäuses 12 umfasst das Gehäuse 12 einen vom übrigen Gehäuse 20 längs der Prismenachse P abnehmbaren und am übrigen Gehäuse 20 anordenbaren Gehäusedeckel 22. Der Gehäusedeckel 22 dient somit zum Verschließen einer an dem dem Betrachter von Figur 1 näher gelegenen Längsende 14e der Mantelfläche 14 gebildeten Gehäuseöffnung 24. Die Gehäuseöffnung 24 ist durch die Mantelfläche 14 des Restgehäuses 20 begrenzt. Durch die Gehäuseöffnung 24 ist ein Filter-Aufnahmefach 26 für eine Luftfilter-Kartusche 28 mit einem Luftfilter 29 und ist ein Akku-Aufnahmefach 30 für einen wiederaufladbaren elektrischen Akkumulator 32 als einen netzunabhängigen Energiespeicher 34 zugänglich.

Der Gehäusedeckel 22 weist ein Deckelbauteil 36 und ein Verriegelungsbauteil 38 auf. Das Verriegelungsbauteil 38 ist am Deckelbauteil 36 um die Verriegelungsachse V drehbar gelagert. Die Verriegelungsachse V verläuft im Verschlusszustand, also dann, wenn der Gehäusedeckel 22 am Restgehäuse 20 angeordnet ist und die Gehäuseöffnung 24 verschließt, koaxial mit der Prismenachse P.

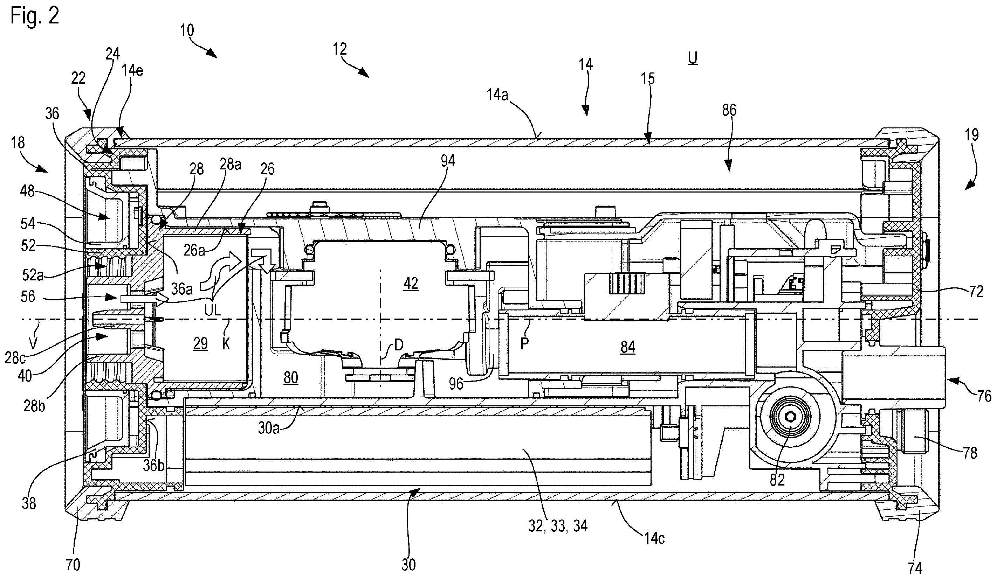

Der Gehäusedeckel 22 weist außerdem eine Umgebungsluft-Ansaugöffnung 40 auf, welche sowohl das Deckelbauteil 36 als auch das Verriegelungsbauteil 38 durchsetzt. Durch die Umgebungsluft-Ansaugöffnung 40 hindurch kann von einem Gebläse 42 (siehe Figur 2) Umgebungsluft aus der Umgebung U durch den Luftfilter 29 hindurch in das Gehäuse 12 angesaugt werden.

Das Verriegelungsbauteil 38 ist in Figur 1 in seiner Verriegelungsstellung gezeigt, von welcher es entgegen dem Uhrzeigersinn etwa um eine Zwölftel-Umdrehung um die Verriegelungsachse V in eine durch ein Symbol 43 in Gestalt eines offenen Vorhängeschlosses gekennzeichnete Freigabestellung drehbar ist. Das Verriegelungsbauteil 38 weist radial in Richtung von der Verriegelungsachse V weg vorstehende Vorsprünge auf, welche in Figur 1 durch das Deckelbauteil 36 verdeckt sind. Diese Vorsprünge sind Teil einer Bajonettverriegelung, durch welche der die Gehäuseöffnung 24 verschließende Gehäusedeckel 22 an einer relativ zum Restgehäuse 20 unbeweglichen Verriegelungsgegenformation 44 formschlüssig verriegelbar ist. Die Verriegelungsgegenformation 44 weist hierzu mehrere Ausnehmungen 46 auf, jeweils mit einem axialen Ausnehmungsabschnitt 46a und mit einem Ausnehmungsabschnitt 46b in Umfangsrichtung um die Verriegelungsachse V. Die Vorsprünge des Verriegelungsbauteils 38 können dann, wenn es sich in der Freigabestellung befindet, längs des axialen Ausnehmungsabschnitts 46a parallel zur Verriegelungsachse V und damit parallel zur Prismenachse P zum Ausnehmungsabschnitt 46b hingeführt werden und nach Erreichen des Ausnehmungsabschnitts 46b in Umfangsrichtung entlang des Ausnehmungsabschnitts 46b bewegt werden.

Das Verriegelungsbauteil 38 weist eine in Umlaufrichtung verlaufende Griffmulde 48 auf, welche durch zwei Griffstege 50a und 50b unterbrochen ist, die einander diametral bezüglich der zwischen ihnen gelegenen Umgebungsluft-Ansaugöffnung 40 gegenüberliegen. Durch Handangriff an die Griffstege 50a und 50b kann sowohl das Verriegelungsbauteil 38 zwischen der Freigabestellung und der Verriegelungsstellung verdreht als auch der freigegebene Gehäusedeckel 22 längs der Prismenachse P vom Restgehäuse 20 abgehoben oder auf dieses aufgesetzt werden. Die Griffstege 50a und 50b sowie die Griffmulde 48 bilden gemeinsam eine Betätigungsformation 51 zur Betätigung des Verriegelungsbauteils 38.

Der Gehäusedeckel 22 ist daher durch einhändige Bedienung sowohl vom Restgehäuse 20 abnehmbar als auch auf dieses aufsetzbar als auch in der Verschlussstellung verriegelbar und freigebbar.

Die Umgebungsluft-Ansaugöffnung 40 ist nach radial außen - bezogen auf die Verriegelungsachse V - unmittelbar durch einen Lagerabschnitt 52 des Deckelbauteils 36 begrenzt. Der Lagerabschnitt 52 weist eine Befestigungsformation 52a in Form eines Innengewindes auf. An dieser Befestigungsformation 52a kann beispielsweise ein zusätzlicher Luftfilter angeordnet werden, welcher Filterfunktionen erfüllt, die der Luftfilter 29 der Luftfilter-Kartusche 28 nicht leistet. Alternativ oder zusätzlich kann an der Befestigungsformation 52a eine Messvorrichtung angeordnet werden, welche die Umgebungsluft-Ansaugöffnung 40 durchströmende angesaugte Umgebungsluft messtechnisch erfasst, etwa deren chemische Zusammensetzung bestimmt oder bestimmt, ob und gegebenenfalls in welchem Ausmaß die angesaugte Umgebungsluft einen vorbestimmten Bestandteil enthält oder nicht.

Der Lagerabschnitt 52 ist radial außen von einem Lagergegenabschnitt 54 des Verriegelungsbauteils 38 umgeben. Der Lagerabschnitt 52 wirkt gleichsam als Achsbauteil, welches das Verriegelungsbauteil 38 mittels dessen Lagergegenabschnitts 54 um die Verriegelungsachse V drehbar lagert. Der Lagergegenabschnitt 54 bildet eine radial innere Grenze der Griffmulde 48.

Die Luftfilter-Kartusche 28 weist auf ihrer im Betrieb dem Gehäusedeckel 22 zugewandten Seite eine Umgebungsluft-Einlassöffnung 56 auf, welche von einem vom Kartuschenhauptkörper 28a auskragend abstehenden Kragen 28b eingefasst ist.

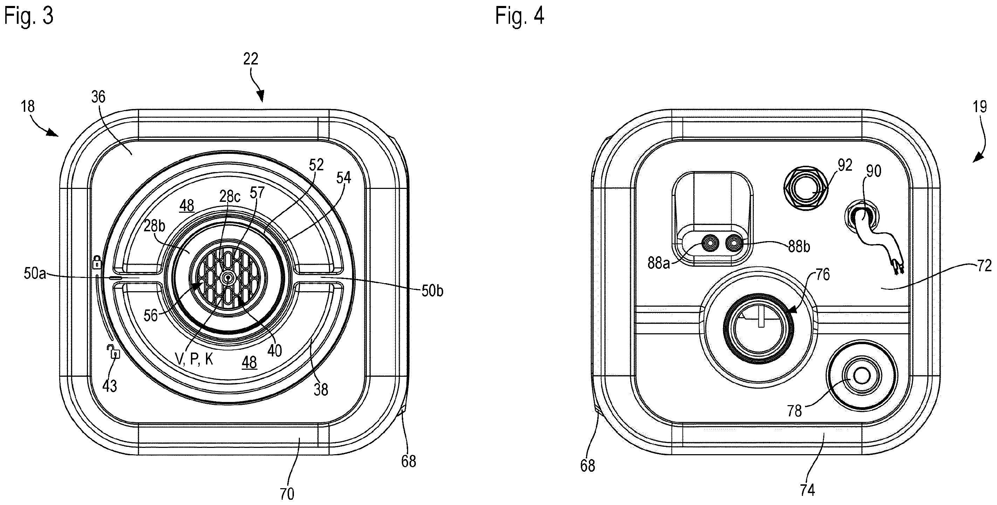

Eine den Kragen 28b zentral durchsetzend gedachte Kartuschen-Einlassachse K ist im betriebsbereiten Zustand des Notfall-Beatmungsgeräts 10 koaxial zur Verriegelungsachse V und zur zentral die Mantelfläche 14 durchsetzend gedachten virtuellen Prismenachse P. Die Umgebungsluft-Einlassöffnung 56 ist durch ein Schutzgitter 57 (siehe Figur 3) vor einem Eintritt größerer Schmutzpartikel, wie Steine, Wollmäuse und dgl., geschützt. Das Schutzgitter 57 kann spritzgusstechnisch einstückig mit einem die Umgebungsluft-Einlasssöffnung aufweisenden Gehäuseteil der Luftfilter-Kartusche 28 ausgebildet sein.

Konzentrisch zu dem Kragen 28b ragt längs der Kartuschen-Einlassachse K ein Sondergas-Hilfseinlass 28c in Gestalt eines sich vom Kartuschenhauptkörper 28a weg verjüngenden auskragenden Anschlussstutzens ab. Eine Sondergasversorgung, beispielsweise eine Sauerstoff-Hilfsversorgung kann an den Sondergas-Hilfseinlass 28c schnell und unkompliziert angeschlossen werden, beispielsweise indem ein in seinem Durchmesser ausreichend klein bzw. groß bemessener elastischer Schlauch auf den Sondergas-Hilfseinlass 28c aufgeschoben und reibschlüssig dort gehalten wird. Durch die sich vom Kartuschenhauptkörper 28a weg verjüngende Gestalt des Sondergas-Hilfseinlasses 28c können Schläuche in einem vorbestimmten Durchmesserbereich ausreichend sicher kurzfristig mit dem Sondergas-Hilfseinlass 28c verbunden werden.

Der Energiespeicher 34 weist in dem dargestellten bevorzugten Ausführungsbeispiel einen einzigen Energiespeicherkörper 33 auf.

Auf dem ebenen Flächenanteil 14d und ausgehend davon sich in die teilzylindrischen benachbarten Flächenanteile 16d und 16a erstreckend weist das Notfall-Beatmungsgerät 10 eine Eingabe-/Ausgabe-Vorrichtung 58 auf, welche dem Informationsaustausch zwischen Bedienperson und Notfall-Beatmungsgerät 10 dient und welche der Steuerung des Notfall-Beatmungsgeräts 10 durch die Bedienperson dient. Die Eingabe-/Ausgabe-Vorrichtung 58 weist einen Bildschirm 60 als eine Ausgabevorrichtung auf, welcher vorzugsweise ein Touchscreen ist, der berührungssensitiv die Eingabe von Information gestattet. Die Eingabe-/Ausgabe-Vorrichtung 58 weist darüber hinaus Anzeige-LEDs 62 als weitere Ausgabevorrichtung auf und weist beispielhaft Tastschalter 64 und einen Drehschalter 66 als Eingabemittel auf.

Zum Schutz vor stoßartigen Belastungen kann die Eingabe-/Ausgabe-Vorrichtung 58 durch ein Fassungsbauteil 67, in beispielhafter Ausgestaltung als stoßdämpfender Elastomerring 68, etwa aus Gummi, Kautschuk und dergleichen, umgeben sein. Das die Eingabe-/Ausgabe-Vorrichtung 58 umgebende Fassungsbauteil 67 kann jedoch auch als Kunststoffspritzgussbauteil aus einem thermoplastischen Kunststoff gebildet sein.

Auch der Gehäusedeckel 22 ist durch einen Umlaufrichtung vollständig um die Prismenachse P umlaufenden stoßdämpfenden Elastomerring 70 umgeben. Im Verschlusszustand bedeckt der Elastomerring 70 ebenso einen Teil der Mantelfläche 14 wie der Stirnseite 18, sodass der Elastomerring 70 im Bereich des Gerätedeckels 22 das Notfall-Beatmungsgerät 10 sowohl gegen axiale als auch gegen radiale Stoßbelastungen schützt.

An dem dem Gehäusedeckel 22 entgegengesetzten Längsende 14f der Mantelfläche 14 ist ebenfalls ein Gerätedeckel 72 (siehe Figur 2) angeordnet. Im Gegensatz zum Gerätedeckel 22 ist der Gerätedeckel 72 jedoch bevorzugt nicht von der Mantelfläche 14 des Gehäuses 12 abnehmbar. Um auch das Längsende des Gerätedeckels 72 vor axialen und radialen Stoßbelastungen zu schützen ist auch an diesem Längsende ein vollständig geschlossen in Umlaufrichtung um die Prismenachse P umlaufender Elastomerring 74 vorgesehen, welcher sowohl einen Teil der Mantelfläche 14 als auch einen Teil der Stirnseite 19 bedeckt. Die Stirnseite 19 ist der Stirnseite 18 entgegengesetzt.

Die Elastomerringe 68, 70 und 74 sind zur fertigungstechnischen Vereinfachung bevorzugt aus demselben weichelastischen Material gebildet.

Figur 2 zeigt einen Längsschnitt durch das Notfall-Beatmungsgerät 10 längs einer Schnittebene, welche die Prismenachse P enthält und parallel zu den ebenen Flächenanteilen 14d und 14b verläuft.

Wie in dem in Figur 2 gezeigten betriebsbereiten Verschlusszustand des Notfall-Beatmungsgeräts 10 erkennbar ist, weist das Deckelbauteil 36 einen Deckel-Filter-Positionierabschnitt 36a auf, welcher im Verschlusszustand in Anlageeingriff ist mit einem Abschnitt der Luftfilter-Kartusche 28, insbesondere mit dem Kartuschenhauptkörper 28a und so zur definierten Lage der Luftfilter-Kartusche 28 und des Luftfilters 29 im Gehäuse 12 beiträgt. Weiter weist das Notfall-Beatmungsgerät 10 einen Gehäuse-Filter-Positionierabschnitt 26a auf, beispielsweise in Gestalt einer Innenwand des Filter-Aufnahmefachs 26. Im Zusammenwirken definieren der Deckel-Filter-Positionierabschnitt 36a und der Gehäuse-Filter-Positionierabschnitt 26a die Betriebsposition der Luftfilter-Kartusche 28 ausreichend genau.

Ebenso weist das Deckelbauteil 36 einen Deckel-Speicher-Positionierabschnitt 36b auf, welche im dargestellten Verschlusszustand in Anlageeingriff ist mit dem Energiespeicherkörper 33 und den Energiespeicherkörper 33 im Zusammenwirken mit einem Gehäuse-Speicher-Positionierabschnitt 30a, etwa einer Innenwand des Akku-Aufnahmefachs 30, ausreichend genau in seiner Betriebsposition fixiert.

Auf der Stirnseite 19 im gehäusefesten Gehäusedeckel 72 liegt die Beatmungsgas-Ausgabeöffnung 76 (siehe auch Figur 4), durch welche hindurch vom Gebläse 42 gefördertes inspiratorisches Beatmungsgas aus dem Gehäuse 12 zu einem an das Notfall-Beatmungsgerät 10 angeschlossenen Patienten hin austritt.

Hinter der Schnittebene von Figur 2, unterhalb der Beatmungsgas-Ausgabeöffnung 76 ist, wiederum im gehäusefesten Gehäusedeckel 72, ein Sondergas-Kopplungsabschnitt 78, etwa ein Sondergas-Anschlussstutzen, vorgesehen, durch welchen ebenfalls ein von Umgebungsluft verschiedenes Sondergas in das Notfall-Beatmungsgerät 10 eingeleitet werden kann. Auch dieses Sondergas kann beispielsweise Sauerstoff sein.

Somit gestattet das Notfall-Beatmungsgerät 10 die Mischung eines Beatmungsgases aus drei verschiedenen Gasen, nämlich aus Umgebungsluft, aus einem durch den Sondergas-Kopplungsabschnitt 78 eingeleiteten ersten Sondergas und aus einem durch den Sondergas-Hilfseinlass 28c eingeleiteten zweiten Sondergas. Sofern nur ein weiteres von Umgebungsluft verschiedenes Sondergas zur Mischung des Beatmungsgases benötigt wird, wird dieses bevorzugt über den Sondergas-Kopplungsabschnitt 78 eingeleitet.

Durch die Umgebungsluft-Ansaugöffnung 40 angesaugte Umgebungsluft UL tritt, wie durch die weiß gefüllten Pfeile in Figur 2 dargestellt, durch die Umgebungsluft-Einlassöffnung 56 in den Kartuschenhauptkörper 28a ein, tritt durch den Luftfilter 29 hindurch und erreicht eine Mischkammer 80, in welcher das Gebläse 42 mit seiner Ansaugöffnung angeordnet ist. Das in der Mischkammer 80 vorhandene Gas benetzt einen Großteil der Außenfläche des Gebläses 42 und trägt so zu dessen konvektiver Kühlung bei.

Ein durch den Sondergas-Kopplungsabschnitt 78 eingeleitetes Sondergas, beispielsweise Sauerstoff, kann in seinem Mengenstrom durch ein verstellbares Proportionalventil 82 über die Eingabe-/Ausgabe-Vorrichtung 58 passend eingestellt werden und erreicht über eine Sondergas-Zufuhrleitung 84 ebenfalls die Mischkammer 80, wo sich die Umgebungsluft UL und dass Sondergas bereits vor Eintritt in das Gebläse 42 vermischen können. Das Gebläse 42 dient vorliegend also nicht nur zur Förderung des Beatmungsgases sondern auch zu dessen möglichst homogener Durchmischung, sodass ein möglichst homogenes Beatmungsgas aus der Beatmungsgas-Ausgabeöffnung 76 austritt. Die Förderleitung, welche druckseitig das Beatmungsgas vom Gebläse 42 zur Beatmungsgas-Ausgabeöffnung 76 leitet, liegt in Figur 2 hinter der Schnittebene der Figur 2 und liegt hinter einem Elektronik-Kompartiment 86, welches gegenüber der Sondergas-Zufuhrleitung 84 körperlich vollständig abgeschirmt ist, um jegliche Zündgefahr auszuschließen, die ein Funke, welcher in der im Elektronik-Kompartiment 86 aufgenommenen Elektronik entstehen könnte, oder auch nur ausreichende Hitze in einer Umgebung reinen Sauerstoffs oder stark erhöhten Sauerstoffgehalts haben könnte. Im Elektronik-Kompartiment 86 ist eine Steuervorrichtung zur Steuerung des Betriebs des Notfall-Beatmungsgeräts 10 aufgenommen.

In Figur 3 ist eine Draufsicht auf die Stirnseite 18 mit dem abnehmbaren Gehäusedeckel 22 dargestellt, also mit Blickrichtung längs der koaxialen Achsen Verriegelungsachse V, Prismenachse P und Kartuschen-Einlassachse K.

Figur 4 zeigt eine Draufsicht auf die Stirnseite 19 mit gehäusefesten Gehäusedeckel 72. Die Blickrichtung von Figur 4 ist jener von Figur 3 entgegengesetzt.

Über die bereits im Zusammenhang mit den Figuren 1 und 2 erläuterten Merkmale hinaus zeigt Figur 4 Anschlussstutzen 88a und 88b, an welche Druckerfassungsschläuche anschließbar sind, welche an ihrem von den Anschlussstutzen 88a bzw. 88b fernliegenden anderen Ende jeweils mit einem Innenbereich eines Differenzdruck-Flusssensors zur Messung eines proximalen inspiratorischen und bevorzugt auch exspiratorischen Beatmungsgasstroms verbunden sind. Die beiden Innenbereiche sind in an sich bekannter Weise durch einen Strömungswiderstand voneinander getrennt, wobei der Strömungswiderstand durch den Beatmungsgasstrom veränderlich ist.

Über einen Netzeingang 90 ist das Notfall-Beatmungsgerät 10 bei räumlicher Verfügbarkeit eines Netzanschlusses mit Energie aus einem öffentlichen Stromversorgungsnetz betreibbar. Alle elektrischen Funktionseinheiten des Notfall-Beatmungsgeräts 10 können dann mit Netzspannung versorgt werden, in der Regel unter Zwischenschaltung eines auf Niedervoltspannung transformierenden Netzteils im Gehäuse 12. Ebenso kann der Akkumulator 32 aufgeladen werden. Eine Buchse 92 im Gehäuse 12 ist zum Anschluss eines externen Sensors, insbesondere CO₂-Sensors, angeordnet. Ein solcher CO₂-Sensor kann beispielsweise an einem mit der Notfall-Beatmungsvorrichtung 10 gekoppelten Durchflusssensor vorgesehen und mit einer Sensoranordnung gekoppelt sein.

In den Schnittansichten der Figuren 2, 5 und 6 ist in Schnittansicht ein Wärmeleitkörper 94 dargestellt, an welchem das Gebläse 42 gehaltert ist.

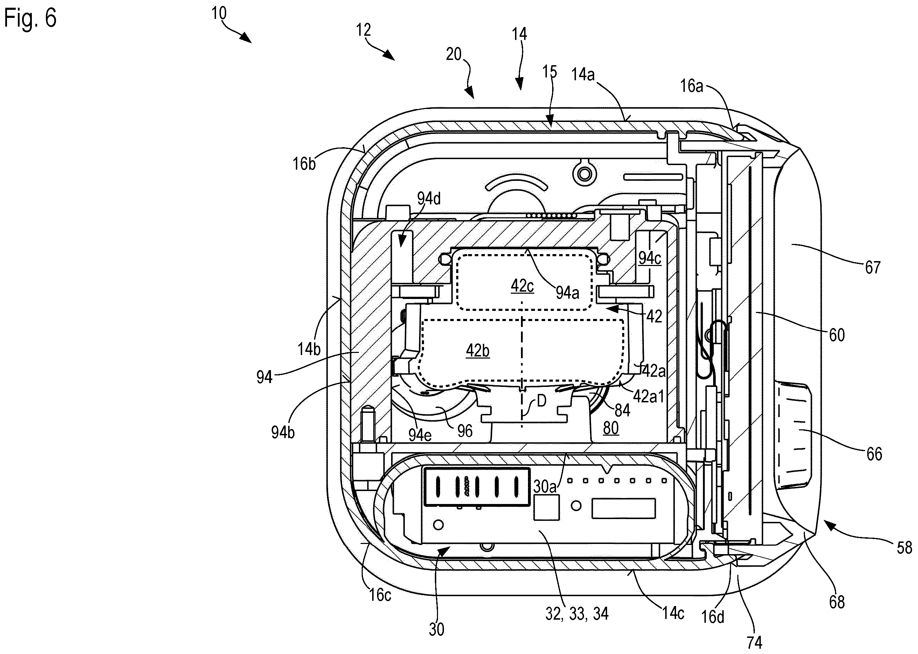

Wie in Figur 6 zu erkennen ist, ist in einem unteren Abschnitt des Gebläsegehäuses 42a ein Luftförderer 42b um eine zum ebenen Flächenanteil 14c orthogonale und zur Zeichenebene der Figuren 2 und 6 parallele Drehachse D drehbar aufgenommen. Ein über dem Luftförderer 42b gelegener elektrischer Antrieb 42c treibt den beispielhaft als Flügelrad ausgebildeten Luftförderer 42b zur Drehung an. Der untere Abschnitt des Gebläsegehäuses 42a kann als gesondertes Fördergehäuseteil ausgebildet sein, aus Kostengründen etwa aus Kunststoff. Das Fördergehäuseteil kann zur Erleichterung der Montage selbst wiederum mehrteilig ausgestaltet sein.

Ein den Antrieb 42c umgebender Teil des Gebläsegehäuses 42a ist in einer von einer Gebläse-Verbindungsfläche 94a mit geringem Spaltmaß von weniger als 1 mm, vorzugsweise von weniger als 0,3 mm, besonders bevorzugt spaltfrei, begrenzten Ausnehmung am Wärmeleitkörper 94 befestigt, beispielsweise durch Kleben oder Löten oder Schweißen oder durch Verbindungsmittel wie Schrauben. Dieser Teil des Gebläsegehäuses 42a kann als gesondertes Antriebsgehäusebauteil ausgebildet sein, zur besseren Wärmeleitung etwa aus einer Aluminium- oder Metalllegierung.

Das bevorzugt aus Aluminium im Druckgussverfahren oder durch spanende Bearbeitung aus dem Vollen hergestellte Gebläsegehäuse 42a überträgt Wärme vom Gebläse 42 zum Wärmeleitkörper 94. Da der Antrieb 42c im Betrieb des Notfall-Beatmungsgeräts 10 innerhalb des Gebläses 42 die bedeutendste Wärmequelle darstellt, umgibt die Gebläse-Verbindungsfläche 94a bevorzugt den den Antrieb 42c einhausenden Bereich des Gebläsegehäuses 42a.

Der ebenfalls bevorzugt aus Aluminium hergestellte Wärmeleitkörper 94 weist mit Abstand von der Gebläse-Verbindungsfläche 94a eine Gehäuse-Verbindungsfläche 94b auf, mit welcher der Wärmeleitkörper 94 vollflächig an der Innenseite des den ebenen Flächenanteil 14b aufweisenden Gehäuseabschnitts anliegend mit dem Gehäuse 12 verbunden ist. Bevorzugt ist der Wärmeleitkörper 94 von außen durch Durchgangslöcher in dem betreffenden Gehäuseabschnitt mit nicht gezeigten Schrauben befestigt. Die Schrauben durchsetzen die Durchgangslöcher und sind in Innengewinde am Wärmeleitkörper 94 eingedreht. So kann die Gehäuse-Verbindungsfläche 94b vollflächig spaltfrei mit dem Gehäuseabschnitt verbunden sein.

Alternativ zur Darstellung in den Figuren 2 und 6 können zwischen der Gebläse-Verbindungsfläche 94a und dem Gebläsegehäuse 42a oder/und zwischen der Gehäuse-Verbindungsfläche 94b und dem Gehäuse 12 die Wärmeleitung erhöhende Zwischenschichten angeordnet sein, beispielsweise als pastöse Schicht einer Wärmeleitpaste oder demgegenüber bevorzugt als Festkörperschicht in Gestalt einer Wärmeleitmatte.

Vom Gebläse 42 an den Wärmeleitkörper 94 übertragene Wärme folgt dem sich im Betrieb ausbildenden Temperaturgradienten am ebenen Flächenanteil 14b, wo in der Regel an der Kontaktfläche zur Außenumgebung U die niedrigste Temperatur im Pfad vom Gebläse 42 über den Wärmeleitkörper 94 bis zum Gehäuse 12 vorliegt. Am Flächenanteil 14b wird die vom Wärmeleitkörper 94 vom Gebläse 42 zum Gehäuse 12 geleitete Wärme an die Außenumgebung U durch Konvektion und Strahlung abgegeben. Eine Konvektionsströmung kann sich aufgrund des Temperaturunterschieds zwischen dem Flächenanteil 14b und der Außenumgebung U natürlich ausbilden und wird umso ausgeprägter sein, je größer der Temperaturunterschied zwischen dem Flächenanteil 14b und der Außentemperatur U ist. Da das die Mantelfläche 14 aufweisende rohrförmige Gehäusebauteil 15 bevorzugt aus dem gut wärmeleitenden Werkstoff Aluminium hergestellt ist, leitet das Gehäusebauteil 15 Wärme von dem Flächenanteil 14b auch zu benachbarten Flächenanteilen 14a, 16b, 16c, 14c, usw., so dass auch solche Flächenanteile zur Abführung von Wärme an die Außenumgebung U beitragen können, die nicht unmittelbar in Berührkontakt mit dem Wärmeleitkörper 94 stehen.

Die Gehäuse-Verbindungsfläche 94b ist mehr als doppelt so groß wie die Gebläse-Verbindungsfläche 94a.

Wie in Figur 6 zu erkennen ist, ragt ein Großteil der Außenfläche 42a1 des Gebläsegehäuses 42a in die Mischkammer 80, wo der einragende Teil der Außenfläche 42a1 von Beatmungsgas in der Mischkammer 80 benetzbar ist. So kann auch das vom Gebläse 42 geförderte Beatmungsgas zur konvektiven Kühlung des Gebläses 42 und insgesamt des Notfall-Beatmungsgeräts 10 beitragen. Die Außenfläche 42a1 umgibt die Drehachse D des Luftförderers 42b in Umfangsrichtung vollständig.

Bevorzugt ist die Kühlwirkung des Beatmungsgases und des Wärmeleitkörpers 94 so gut, dass das Notfall-Beatmungsgerät 10 keinen dedizierten Kühlerlüfter aufweist, sodass bevorzugt das Gebläse 42 zur Förderung von Beatmungsgas das einzige Gebläse im Notfall-Beatmungsgerät 10 ist.

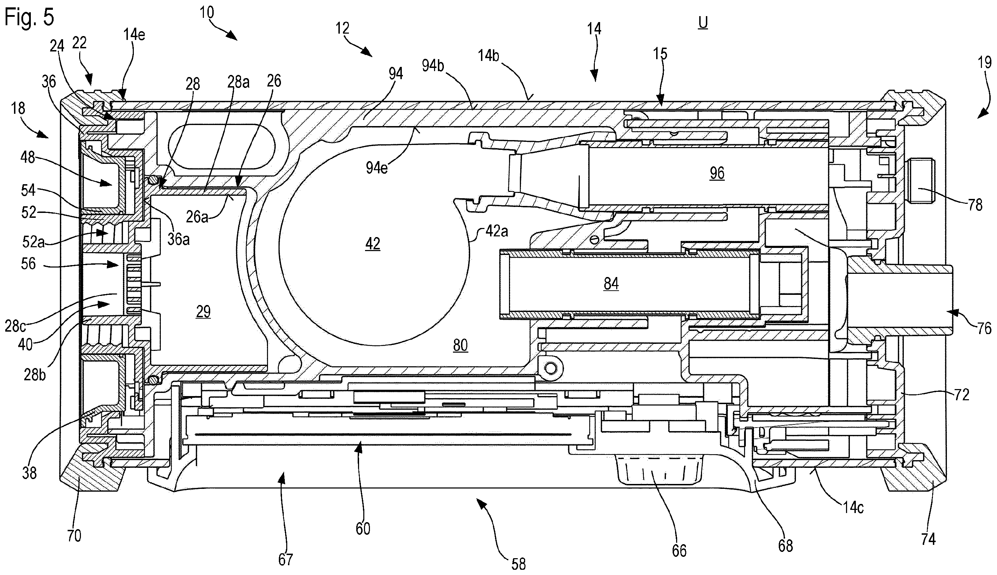

In Figur 5 ist ein Beatmungsgaskanal 96 als Ausgangskanal des Gebläses 42 zu erkennen. Auf der Druckseite des Gebläses 42 fördert das Gebläse 42 Beatmungsgas durch den Beatmungsgaskanal 96 in Richtung zur Beatmungsgas-Ausgabeöffnung 76. Der Beatmungsgaskanal 96 verläuft im dargestellten Ausführungsbeispiel platzsparend parallel zur Sondergas-Zuführleitung 84.

Im Wärmeleitkörper 94 können die Oberfläche des Wärmeleitkörpers 94 erhöhende Kanäle 94c und 94d ausgebildet sein, welche, angetrieben durch das Gebläse 42, wenigstens abschnittsweise von Beatmungsgas in der Mischkammer 80 durchströmt werden können und so zusätzlich Wärme vom Wärmeleitkörper 94 konvektiv abtransportieren. Dies erhöht die Kühlwirkung des Beatmungsgases und des Wärmeleitkörpers 94 zusätzlich.

Eine Oberfläche 94e des Wärmeleitkörpers begrenzt die Mischkammer 80 und ist von Beatmungsgas benetzbar.

Wie in Zusammenschau vor allem der Figuren 5 und 6 zu erkennen ist, umgibt der einstückige Wärmeleitkörper 94 die Mischkammer 80 von fünf Seiten. In der Mischkammer 80 sind der Luftförderer 42b und der den Luftförderer 42b umgebende Teil des Gebläsegehäuses 42a angeordnet. Der in die Mischkammer 80 einragende Teil des Gebläses 42 ist allseits mit Abstand vom Wärmeleitkörper 94 angeordnet, um eine möglichst große Fläche zu erzielen, welche Wärme an das Beatmungsgas in der Mischkammer 80 abgeben kann.

## Patentansprüche

1. Notfall-Beatmungsgerät (10) zur notfallmedizinischen künstlichen Beatmung von Patienten, umfassend
- ein Gehäuse (12) mit einer Umgebungsluft-Ansaugöffnung (40) und einer Beatmungsgas-Ausgabeöffnung (76),
- ein Gebläse (42), welches dazu ausgebildet und im Gehäuse (24) angeordnet ist, um Umgebungsluft von der Umgebungsluft-Ansaugöffnung (40) zur Beatmungsgas-Ausgabeöffnung (76) zu fördern,
- einen Luftfilter (29), welcher zur Reinigung von angesaugter Umgebungsluft ausgebildet ist und im Gehäuse (12) im Strömungsweg der Umgebungsluft stromabwärts der Umgebungsluft-Ansaugöffnung (40) angeordnet ist, und
- einem Energiespeicher (34) zur Versorgung des Gebläses mit Energie für dessen Betrieb,
wobei der Luftfilter (29) durch eine mittels eines Gehäusedeckels (22) verschlossene, aber öffenbare Gehäuseöffnung (24) zugänglich und bestimmungsgemäß austauschbar im Gehäuse (12) aufgenommen ist, und
wobei der Energiespeicher (34) durch eine mittels eines Gehäusedeckels (22) verschlossene, aber öffenbare Gehäuseöffnung (24) zugänglich und bestimmungsgemäß austauschbar im Gehäuse (12) aufgenommen ist,
**dadurch gekennzeichnet, dass** der Luftfilter (29) und der Energiespeicher (34) durch eine gemeinsame Gehäuseöffnung (24) zugänglich sind, wobei die gemeinsame Gehäuseöffnung (24) durch einen gemeinsamen Gehäusedeckel (22) wahlweise verschließbar und öffenbar ist.

2. Notfall-Beatmungsgerät (10) nach Anspruch 1,
**dadurch gekennzeichnet, dass** der gemeinsame Gehäusedeckel (22) ein im Verschlusszustand des Notfall-Beatmungsgeräts, in welchem der gemeinsame Gehäusedeckel (22) die gemeinsame Gehäuseöffnung (24) verschließt, relativ zum übrigen Gehäuse (20) unbewegliches Deckelbauteil (36) und ein relativ zum übrigen Gehäuse (20) bewegliches Verriegelungsbauteil (38) aufweist, wobei das Verriegelungsbauteil (38) beweglich ist zwischen einer Verriegelungsstellung, in welcher eine Verriegelungsformation des Verriegelungsbauteils (38) durch Formschlusseingriff mit einer gehäusefesten Verriegelungsgegenformation (44) am übrigen Beatmungsgerät (10) den gemeinsamen Gehäusedeckel (22) gegen ein Entfernen von der gemeinsamen Gehäuseöffnung (24) verriegelt, und einer Freigabestellung, in welcher das Verriegelungsbauteil (38) ein Entfernen des gemeinsamen Gehäusedeckels (22) von der gemeinsamen Gehäuseöffnung (24) gestattet.

3. Notfall-Beatmungsgerät (10) nach Anspruch 2,
**dadurch gekennzeichnet, dass** das Verriegelungsbauteil (38) relativ zum Deckelbauteil (36) um eine Verriegelungsachse (V) drehbar am Deckelbauteil (36) gelagert ist.

4. Notfall-Beatmungsgerät (10) nach einem der vorhergehenden Ansprüche,
**dadurch gekennzeichnet, dass** der gemeinsame Gehäusedeckel (22) die Umgebungsluft-Ansaugöffnung (40) aufweist.

5. Notfall-Beatmungsgerät (10) nach Anspruch 4, unter Rückbeziehung auf Anspruch 2 oder 3
**dadurch gekennzeichnet, dass** die Umgebungsluft-Ansaugöffnung (40) das Verriegelungsbauteil (38) durchsetzt.

6. Notfall-Beatmungsgerät (10) nach Anspruch 5, unter Einbeziehung des Anspruchs 3,
**dadurch gekennzeichnet, dass** die Verriegelungsachse (V) die Umgebungsluft-Ansaugöffnung (40) durchsetzt.

7. Notfall-Beatmungsgerät (10) nach Anspruch 6,
**dadurch gekennzeichnet, dass** das Deckelbauteil (36) einen zur Verriegelungsachse (V) koaxialen Lagerabschnitt (52) aufweist, welcher die Umgebungsluft-Ansaugöffnung (40) umgibt, und welcher von einem sich längs der Verriegelungsachse (V) erstreckenden und zur Verriegelungsachse (V) koaxialen Lagergegenabschnitt (54) des Verriegelungsbauteils (38) umgeben ist.

8. Notfall-Beatmungsgerät (10) nach Anspruch 7,
**dadurch gekennzeichnet, dass** der Lagerabschnitt (52), auf seiner von dem Lagergegenabschnitt (44) wegweisenden, bezüglich der Verriegelungsachse (V) radial inneren Seite ein Befestigungsformation (52a) aufweist.

9. Notfall-Beatmungsgerät (10) nach einem der vorhergehenden Ansprüche,
**dadurch gekennzeichnet, dass** der gemeinsame Gehäusedeckel einen im Verschlusszustand in das Innere des Gehäuses (12) weisenden Deckel-Filter-Positionierabschnitt (36a) aufweist, welcher im Verschlusszustand bei betriebsbereitem Notfall-Beatmungsgerät (10) im Zusammenwirken mit wenigstens einem gehäusefesten Gehäuse-Filter-Positionierabschnitt (26a) eine Filterkartusche (28) zur Filterung von Umgebungsluft in ihrer betriebsbereiten Position sichert,
oder/und
dass der gemeinsame Gehäusedeckel (22) einen im Verschlusszustand in das Innere des Gehäuses (12) weisenden Deckel-Speicher-Positionierabschnitt (36b) aufweist, welcher im Verschlusszustand bei betriebsbereitem Notfall-Beatmungsgerät (10) im Zusammenwirken mit wenigstens einem gehäusefesten Gehäuse-Speicher-Positionierabschnitt (30a) einen Energiespeicherkörper (33) in seiner betriebsbereiten Position sichert.

10. Notfall-Beatmungsgerät (10) nach Anspruch 9,
**dadurch gekennzeichnet, dass** die Filterkartusche (28) eine durch die Umgebungsluft-Ansaugöffnung (40) erreichbare Umgebungsluft-Einlassöffnung (56) und eine Sondergas-Anschlussformation (28c) zum Anschluss einer Sondergasversorgung aufweist.

11. Notfall-Beatmungsgerät (10) nach Anspruch 9,
**dadurch gekennzeichnet, dass** die Umgebungsluft-Einlassöffnung (56) und die Sondergas-Anschlussformation (28c) zueinander koaxial angeordnet sind, vorzugsweise im betriebsbereiten Verschlusszustand auch koaxial zur Verriegelungsachse (V) angeordnet sind.

12. Notfall-Beatmungsgerät (10) nach einem der vorhergehenden Ansprüche,
**dadurch gekennzeichnet, dass** das Gehäuse (12) eine prismatische oder/und zylindrische Grundform aufweist, wobei der gemeinsame Gehäusedeckel (22) eine Stirnseite (18) des prismatischen oder/und zylindrischen Gehäuses (12) bildet.

13. Notfall-Beatmungsgerät (10) nach Anspruch 12,
**dadurch gekennzeichnet, dass** alle ein Gas in das Gehäuse (12) einleitenden und Gas aus dem Gehäuse (12) ausleitenden Anschlussformationen oder/und Öffnungen (54) an einer der Stirnseiten (18, 19) des prismatischen oder/und zylindrischen Gehäuses (12) angeordnet sind.

14. Notfall-Beatmungsgerät (10) nach Anspruch 12 oder 13,
**dadurch gekennzeichnet, dass** das prismatische oder/und zylindrische Gehäuse (12) im Bereich seiner Mantelfläche (14) eine Eingabe-/AusgabeVorrichtung (58) mit einer Anzeigevorrichtung (60) und wenigstens einer Schaltvorrichtung (64, 66) aufweist.

15. Notfall-Beatmungsgerät (10) nach einem der vorhergehenden Ansprüche,
**dadurch gekennzeichnet, dass** der Gehäusedeckel (22) an seiner Außenfläche wenigstens ein stoßdämpfendes Element (68, 70, 74) aufweist.

## Claims

1. Emergency ventilator (10) for emergency medicine artificial respiration of patients, comprising
- A housing (12) with an ambient air aspiration aperture (40) and a respiratory gas output aperture (76),
- A fan (42) which is configured and arranged in the housing (12) in order to convey ambient air from the ambient air aspiration aperture (40) to the respiratory gas output aperture (76),
- An air filter (29) which is configured for cleaning aspirated ambient air and is arranged in the housing (12) in the flow path of the ambient air downstream of the ambient air aspiration aperture (40), and
- An energy store (34) for supplying the fan with energy for its operation,
Where the air filter (29) is accessible through a housing aperture (24) which is closed by means of a housing lid (22) but is openable and in normal operation is accommodated in the housing (12) in a replaceable manner, and
Where the energy store (34) is accessible through a housing aperture (24) which is closed by means of a housing lid (22) but is openable and in normal operation is accommodated in the housing (12) in a replaceable manner,
**Characterized in that** the air filter (29) and the energy store (34) are accessible through a common housing aperture (24), where the common housing aperture (24) can be closed and opened selectively through a common housing lid (22).

2. Emergency ventilator (10) according to Claim 1,
**Characterized in that** the common housing lid (22) exhibits a lid component (36) which in the closure state of the emergency ventilator, in which the common housing lid (22) closes the common housing aperture (24), is immoveable relative to the remaining housing (20), and a latching component (38) which is moveable relative to the remaining housing (20), where the latching component (38) is moveable between a latching position in which a latching formation of the latching component (38), through positive-locking engagement with a housing-fixed latching counter-formation (44) on the remaining ventilator (10), latches the common housing lid (22) against removal from the common housing aperture (24), and a releasing position in which the latching component (38) allows removal of the common housing lid (22) from the common housing aperture (24).

3. Emergency ventilator (10) according to Claim 2,
**Characterized in that** the latching component (38) is mounted on the lid component (36) rotatably about a latching axis (V) relative to the lid component (36).

4. Emergency ventilator (10) according to one of the preceding Claims,
**Characterized in that** the common housing lid (22) exhibits the ambient air aspiration aperture (40).

5. Emergency ventilator (10) according to Claim 4, by reference to Claim 2 or 3
**Characterized in that** the ambient air aspiration aperture (40) penetrates through the latching component (38).

6. Emergency ventilator (10) according to Claim 5, in consideration of Claim 3,
**Characterized in that** the latching axis (V) penetrates through the ambient air aspiration aperture (40).

7. Emergency ventilator (10) according to Claim 6,
**Characterized in that** the lid component (36) exhibits a mounting section (52) surrounding the ambient air aspiration aperture (40) which is coaxial to the latching axis (V) and which is surrounded by a mounting counter-section (54) of the latching component (38) which extends along the latching axis (V) and is coaxial to the latching axis (V).

8. Emergency ventilator (10) according to Claim 7,
**Characterized in that** the mounting section (52) exhibits an attachment formation (52a) on its relative to the latching axis (V) radial inner side which faces away from the mounting counter-section (44).

9. Emergency ventilator (10) according to one of the preceding Claims,
**Characterized in that** the common housing lid exhibits a lid-filter positioning section (36a) which in the closure state faces towards the interior of the housing (12) and which in the closure state with a ready for operation emergency ventilator (10), in cooperation with at least one housing-fixed housing-filter positioning section (26a), secures a filter cartridge (28) for filtering ambient air in its ready for operation position,
and/or
That the common housing lid (22) exhibits a lid-store positioning section (36b) which in the closure state faces towards the interior of the housing (12) and which in the closure state with a ready for operation emergency ventilator (10), in cooperation with at least one housing-fixed housing-store positioning section (30a), secures an energy store body (33) in its ready for operation position.

10. Emergency ventilator (10) according to Claim 9,
**Characterized in that** the filter cartridge (28) exhibits an ambient air inlet aperture (56) which can be reached through the ambient air aspiration aperture (40) and a special gas connector formation (28c) for connecting a special gas supply.

11. Emergency ventilator (10) according to Claim 9,
**Characterized in that** the ambient air inlet aperture (56) and the special gas connector formation (28c) are arranged coaxially to one another, preferably in the ready for operation closure state are also arranged coaxially to the latching axis (V).

12. Emergency ventilator (10) according to one of the preceding Claims,
**Characterized in that** the housing (12) exhibits a prismatic and/or cylindrical basic form, where the common housing lid (22) forms an end face (18) of the prismatic and/or cylindrical housing (12).

13. Emergency ventilator (10) according to Claim 12,
**Characterized in that** all connector formations and/or apertures (54) which introduce a gas into the housing (12) and channel gas out of the housing (12) are arranged at one of the end faces (18, 19) of the prismatic and/or cylindrical housing (12).

14. Emergency ventilator (10) according to Claim 12 or 13,
**Characterized in that** the prismatic and/or cylindrical housing (12) exhibits in the region of its lateral surface (14) an input/output device (58) with a display device (60) and at least one switching device (64, 66).

15. Emergency ventilator (10) according to one of the preceding Claims,
**Characterized in that** the housing lid (22) exhibits on its outer surface at least one shock-absorbing element (68, 70, 74).

## Revendications

1. Respirateur d'urgence (10) pour la ventilation artificielle de patients en cas d'urgence médicale, comprenant
- un boîtier (12) avec une ouverture d'aspiration d'air ambiant (40) et une ouverture de sortie de gaz respiratoire (76),
- un ventilateur (42) qui est conçu et disposé dans le boîtier (24) pour transporter l'air ambiant de l'ouverture d'aspiration d'air ambiant (40) vers l'ouverture de sortie de gaz de ventilation (76),
- un filtre à air (29) qui est conçu pour purifier l'air ambiant aspiré et qui est disposé dans le boîtier (12) dans le trajet d'écoulement de l'air ambiant en aval de l'ouverture d'aspiration d'air ambiant (40), et
- un dispositif de stockage d'énergie (34) pour alimenter le ventilateur en énergie pour son fonctionnement,
le filtre à air (29) étant accessible par une ouverture de boîtier (24) fermée au moyen d'un couvercle de boîtier (22) mais pouvant être ouverte, et étant reçu dans le boîtier (12) de manière à pouvoir être remplacé conformément à sa destination, et
le dispositif de stockage d'énergie (34) étant accessible par une ouverture de boîtier (24) fermée au moyen d'un couvercle de boîtier (22) mais pouvant être ouverte, et étant reçu de manière à pouvoir être remplacé dans le boîtier (12) conformément à sa destination,
**caractérisé en ce que** le filtre à air (29) et le dispositif de stockage d'énergie (34) sont accessibles par une ouverture commune (24) du boîtier, l'ouverture commune (24) du boîtier pouvant être fermée et ouverte au choix par un couvercle commun (22) du boîtier.

2. Respirateur d'urgence (10) selon la revendication 1,
**caractérisé en ce que** le couvercle commun (22) du boîtier comporte un élément de couvercle (36) qui, dans un état de fermeture dans lequel le couvercle commun (22) ferme l'ouverture commune (24) du boîtier, est immobile par rapport au reste du boîtier (20), et un élément de verrouillage (38) mobile par rapport au reste du boîtier (20), l'élément de verrouillage (38) étant mobile entre une position de verrouillage, dans laquelle une formation de verrouillage de l'élément de verrouillage (38) par engagement par complément de forme avec une contre-formation de verrouillage (44) solidaire du boîtier sur le reste de l'appareil respiratoire (10) verrouille le couvercle commun (22) contre une démontage de l'ouverture commune (24) du boîtier, et une position de déverrouillage dans laquelle l'élément de verrouillage (38) permet au couvercle commun (22) du boîtier d'être retiré de l'ouverture commune (24) du boîtier.

3. Respirateur d'urgence (10) selon la revendication 2,
**caractérisé en ce que** l'élément de verrouillage (38) est monté sur l'élément de couvercle (36) de manière à pouvoir tourner par rapport à l'élément de couvercle (36) autour d'un axe de verrouillage (V).

4. Respirateur d'urgence (10) selon l'une des revendications précédentes,
**caractérisé en ce que** le couvercle commun (22) du boîtier comporte l'ouverture d'aspiration d'air ambiant (40).

5. Respirateur d'urgence (10) selon la revendication 4, en référence à la revendication 2 ou 3
**caractérisé en ce que** l'ouverture d'aspiration d'air ambiant (40) traverse l'élément de verrouillage (38).

6. Respirateur d'urgence (10) selon la revendication 5, en incluant la revendication 3,
**caractérisé en ce que** l'axe de verrouillage (V) traverse l'ouverture d'aspiration d'air ambiant (40).

7. Respirateur d'urgence (10) selon la revendication 6,
**caractérisé en ce que** l'élément de couvercle (36) présente une partie de palier (52) coaxiale à l'axe de verrouillage (V), qui entoure l'ouverture d'aspiration d'air ambiant (40) et qui est entourée par une partie de contre-palier (54) de l'élément de verrouillage (38) s'étendant le long de l'axe de verrouillage (V) et coaxiale à l'axe de verrouillage (V).

8. Respirateur d'urgence (10) selon la revendication 7,
**caractérisé en ce que** la partie de palier (52) présente, sur son côté opposé à la partie de contre-palier (44) et radialement intérieur par rapport à l'axe de verrouillage (V), une formation de fixation (52a).

9. Respirateur d'urgence (10) selon l'une des revendications précédentes,
**caractérisé en ce que** le couvercle commun du boîtier comporte une partie de positionnement filtre couvercle (52a) qui, à l'état fermé, est orientée vers l'intérieur du boîtier (12) qui, à l'état fermé, lorsque le respirateur d'urgence (10) est prêt à l'emploi, en coopération avec au moins une partie de positionnement boîtier filtre (26a) solidaire du boîtier, maintient une cartouche filtrante (28) destinée à filtrer l'air ambiant dans sa position d'emploi,
et/ou
que le couvercle commun (22) du boîtier comporte une partie de positionnement couvercle stockage (36b) qui, à l'état fermé, est orientée vers l'intérieur du boîtier (12) qui, à l'état fermé, lorsque le respirateur d'urgence (10) est prêt à l'emploi, en coopération avec au moins une partie de positionnement boîtier stockage (30a) solidaire du boîtier, maintient un corps de stockage d'énergie (33) dans sa position d'emploi.

10. Respirateur d'urgence (10) selon la revendication 9,
**caractérisé en ce que** la cartouche filtrante (28) comporte une ouverture d'entrée d'air ambiant (56) accessible par l'ouverture d'aspiration d'air ambiant (40) et une formation de raccordement de gaz spécial (28c) pour le raccordement d'une alimentation en gaz spécial.

11. Respirateur d'urgence (10) selon la revendication 9,
**caractérisé en ce que** l'ouverture d'entrée d'air ambiant (56) et la formation de raccordement de gaz spécial (28c) sont disposées coaxialement l'une par rapport à l'autre, de préférence également coaxialement à l'axe de verrouillage (V) à l'état de fermeture prêt à l'emploi.

12. Respirateur d'urgence (10) selon l'une des revendications précédentes,
**caractérisé en ce que** le boîtier (12) présente une forme de base prismatique et/ou cylindrique, le couvercle commun (22) du boîtier formant une face frontale (18) du boîtier prismatique et/ou cylindrique (12).

13. Respirateur d'urgence (10) selon la revendication 12,
**caractérisé en ce que** toutes les formations de raccordement et/ou ouvertures (54) qui introduisent un gaz dans le boîtier (12) et évacuent le gaz hors du boîtier (12) sont disposées sur l'une des faces frontales (18, 19) du boîtier prismatique et/ou cylindrique (12).

14. Respirateur d'urgence (10) selon la revendication 12 ou 13,
**caractérisé en ce que** le boîtier prismatique et/ou cylindrique (12) comporte, dans la zone de sa surface d'enveloppe (14), un dispositif d'entrée/dépense (58) avec un dispositif d'affichage (60) et au moins un dispositif de commutation (64, 66).

15. Respirateur d'urgence (10) selon l'une des revendications précédentes,
**caractérisé en ce que** le couvercle du boîtier (22) comporte au moins un élément amortisseur de chocs (68, 70, 74) sur sa surface extérieure.
